# EUROPEAN PATENT SPECIFICATION

(11) **EP 2 513 829 B1**
(45) Date of publication and mention of the grant of the patent: **07.04.2021**
(21) Application number: 10800817.8
(22) Date of filing: 15.12.2010
(51) Int. Cl.: G16H 40/00, G16H 80/00, G16H 40/63, G16H 40/40

(54) **SYSTEM AND METHOD TO AUTHORIZE RESTRICTED FUNCTIONALITY**
SYSTEM UND VERFAHREN ZUR AUTORISIERUNG VON EINGESCHRÄNKTER FUNKTIONALITÄT
SYSTÈME ET PROCÉDÉ POUR AUTORISER UNE FONCTIONNALITÉ LIMITÉE

(30) Priority: 16.12.2009 US 287012 P
(43) Date of publication of application: 24.10.2012
(73) Proprietor: Cardiac Pacemakers, Inc., St. Paul, Minnesota 55112 (US)
(72) Inventor: STUBBS, Scott R., Maple Grove, Minnesota 55369 (US); DOSHI, Hiten J., Plymouth, Minnesota 55446 (US)
(74) Representative: Peterreins Schley
(86) International application number: PCT/US2010/060561
(87) International publication number: WO 2011/084510

(56) References cited:
- WO-A1-01/48676
- WO-A1-2006/041827
- WO-A1-2007/041615
- WO-A1-2008/121889
- WO-A2-2007/081829
- US-A1- 2006 190 051

## Description

### TECHNICAL FIELD

This disclosure relates generally to medical devices and, more particularly, to medical systems and methods for controlling authorization of restricted functionality, amongst other things.

### BACKGROUND OF THE INVENTION

Implantable medical devices are commonly used to treat patients with various conditions. By way of example, conditions of the heart such as tachycardia, bradycardia, and heart failure, amongst others, are now routinely treated using implantable cardiac rhythm management (CRM) devices. In addition, various conditions such as epilepsy, chronic pain, Parkinson's disease, and depression can now be treated using implantable neurostimulation devices. Patients with such implantable devices may periodically visit clinicians for follow-up appointments. During these follow-up appointments, clinicians may perform programming tasks such as adjusting the therapy settings of the implanted device in addition to accessing and reviewing data gathered by the device. In order to perform these tasks, the clinician will typically use an external device, such as a programmer or programmer-recorder-monitor (PRM) device in order to interrogate the implanted medical device and reprogram it if necessary.

Published U.S. application US 2006/0190051 A1 discloses an implantable neurostimulator and system capable of providing adaptive neurostimulation therapy to alleviate incontinence. The neurostimulator operates according to a set of stimulation parameters stored in memory. During operation, information is obtained from the patient, the implanted neurostimulator, one or more implanted sensors, or some combination thereof. A processor analyzes the information to automatically generate proposed adjustments to the stimulation parameters applied by the neurostimulator. The adjustments provide an adaptive neurostimulation therapy that supports or enhances therapeutic efficacy based on the information.

Published international application WO 2008/121889 discloses a system and method for confirming identity and authority by a patient medical device. Master credentials are issued to a requesting device and a receiving device from an authorizing agent. The master credentials include the authorizing agent's public key and a digital signature of a root certification authority. Device credentials are issued to the requesting device from the authorizing agent. The device credentials include the requesting device's public key and the authorizing agent's digital signature. Identification credentials are provided to the receiving device and include the device credentials and the requesting device's digital signature. The requesting device is authenticated. The authorizing agent's digital signature in the device credentials is checked using the authorizing agent's public key in the master credentials of the receiving device. The requesting device's digital signature in the identification credentials is checked using the requesting device's public key in the device credentials.

### SUMMARY OF THE INVENTION

The present invention relates to a medical system as set out in claim 1 and a method for restricting programming instructions delivered to an implantable medical device as set out in claim 11. Other embodiments are described in the dependent claims. Embodiments of the invention are related to medical systems and methods for controlling authorization of restricted functionality, amongst other things.

This summary is an overview of some of the teachings of the present application and is not intended to be an exclusive or exhaustive treatment of the present subject matter. Further details are found in the detailed description and appended claims. Other aspects will be apparent to persons skilled in the art upon reading and understanding the following detailed description and viewing the drawings that form a part thereof, each of which is not to be taken in a limiting sense. The scope of the present invention is defined by the appended claims and their legal equivalents.

### BRIEF DESCRIPTION OF THE DRAWINGS

The invention may be more completely understood in connection with the following drawings, in which:
FIG. 1 is a schematic diagram of a system, consistent with at least one embodiment of the technology disclosed herein.
FIG. 2 is a flow diagram in accordance with at least one embodiment of the technology disclosed herein.
FIG. 3 is a flow diagram in accordance with at least one embodiment of the technology disclosed herein.
FIG. 4 is a flow diagram in accordance with at least one embodiment of the technology disclosed herein.
FIG. 5 is a schematic diagram of various programmer device components shown in accordance with at least one embodiment of the technology disclosed herein.
FIG. 6 is a schematic diagram of various implantable medical device components shown in accordance with at least one embodiment of the technology disclosed herein.

While the invention is susceptible to various modifications and alternative forms, specifics thereof have been shown by way of example and drawings, and will be described in detail. It should be understood, however, that the invention is not limited to the particular embodiments described.

### DETAILED DESCRIPTION

Patients with implantable devices that deliver therapy may periodically visit clinicians for follow-up appointments. During these follow-up appointments, clinicians commonly perform tasks such as adjusting the therapy settings of the implanted device in addition to accessing and reviewing data gathered by the device. In order to perform these tasks, the clinician will typically use an external device, such as a programmer or a programmer- recorder-monitor (PRM) device in order to interrogate the implanted medical device and send instructions to it for reprogramming if necessary.

However, it may occasionally become necessary to take steps that are not routine. For example, it may become necessary to update the firmware of a device or to trigger a system reset. While it is important to provide a mechanism through which these steps can take place, it is also important to maintain control over how and when these types of extraordinary steps are taken and to prevent system users from undertaking such steps accidentally. Embodiments of the invention can help avoid possible issues by controlling how restricted system functionality can be engaged and/or implemented. As such, embodiments of the invention can enable certain non-typical steps to be taken when necessary, while maintaining control over the same, thereby ensuring patient safety.

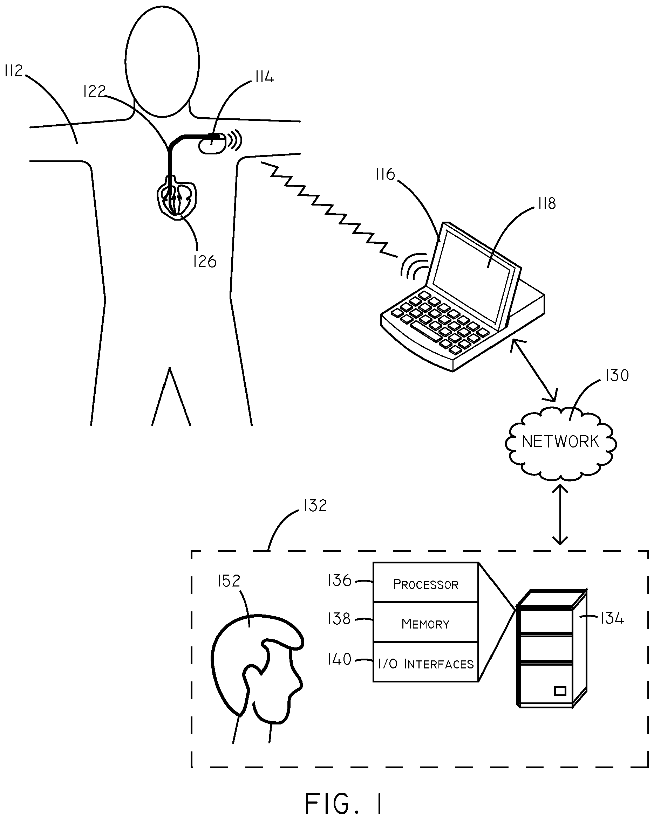

FIG. 1 is a schematic diagram of an example implementation, consistent with at least one embodiment of the invention. An implantable medical device 114 is disposed within a patient 112. The implantable medical device 114 can include one or more stimulation leads 122 that are positioned within proximity of the patient's heart 126.

The implantable medical device 114 can be in communication with an external medical device programmer 116. In some embodiments, the implantable medical device 114 can be in wireless communication with the external medical device programmer 116. Various types of wireless technologies can be used in order to facilitate communication between the external medical device programmer 116 and the implantable medical device 114. By way of example, communication can be carried out through induction, radiofrequency (RF) transmission, acoustically, or the like.

The implantable medical device 114 can be configured to gather data and transmit data to the external medical device programmer 116. For example, the implantable medical device 114 can include sensors and gather data such as heart rate data, electrogram data, pressure data, volume data, flow rate data, temperature data, chemical analyte data, activity data, and/or accelerometer data. The implantable medical device 114 can transmit real-time data to the external medical device programmer 116. In some embodiments, the external medical device programmer 116 can display the real-time data through a video output 118.

The external medical device programmer 116 can also send instructions to the implantable medical device 114 in order to control its functioning. The instructions can be selected from a set of possible instructions. The set of instructions can include restricted and non-restricted instructions. Restricted instructions can include those for which authorization is required before implementation on the implantable medical device 114. Non-restricted instructions can include those for which authorization is not required before implementation on the implantable medical device 114. Examples of restricted and non-restricted instructions are provided in greater detail below. Further, the external medical device programmer 116 can receive input from a system user regarding which instructions from the set of instructions are to be delivered to the specific implanted medical device.

The external medical device programmer 116 can be any type of device or combination of devices capable of sending instructions to the implantable medical device 114. In many embodiments, the external medical device programmer 116 is a programmer/recorder/monitor device. Exemplary programmer/recorder/monitor devices include the Model 3120 Programmer available from Boston Scientific Corporation, Natick, MA. However, it will be appreciated that the external medical device programmer 116 can also take on other forms such as an in-home monitoring system or advanced patient management system. An exemplary in-home monitoring system is the LATITUDE® advanced patient management system, available from Boston Scientific Corporation, Natick, MA. Aspects of exemplary in-home monitoring systems are described in U.S. Pat. No. 6,978,182.

The external medical device programmer 116 can be in communication with a communication network 130. The communication network 130 can be of various types such as a LAN, WAN, a telephonic network, the Internet, or the like. Through the communication network 130, the external medical device programmer 116 can be in communication with a remote key authority 132 either directly or indirectly. In some embodiments, communication through the communication network 130 involves the intervention of a human. In other embodiments, communication through the communication network 130 is completely automated.

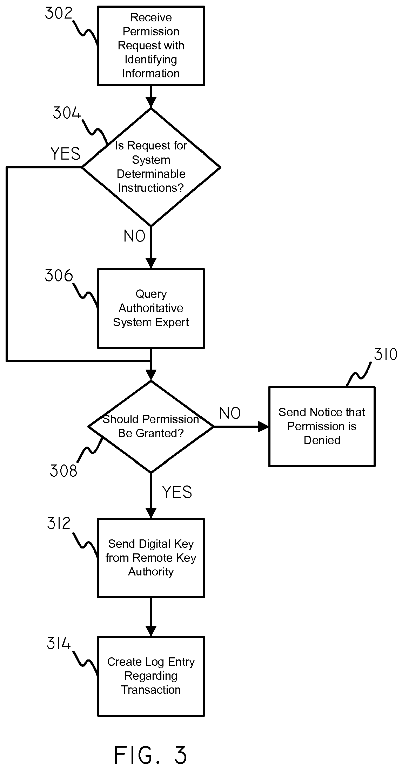

The external medical device programmer 116 can be configured to request permission from the remote key authority 132 if the user input directs delivery of restricted instructions to the implanted medical device 114. In some embodiments, the external medical device programmer 116 can also be configured to initiate a transfer of verifying data to the remote key authority 132 in the context of requesting permission if the user input directs delivery of restricted instructions to the implanted medical device 114. The verifying data can include information specific to the particular implanted medical device 114 in question and/or specific to the patient into which the medical device is implanted. For example, the verifying data could include a patient name, a patient identifying number, a device serial number, or the like. The remote key authority 132 can include various components. By way of example, the remote key authority 132 can include a server 134. The server 134 can include components such as a processor 136, a memory 138, input/output interfaces 140, and the like. The memory can have instructions stored thereon causing the remote key authority to execute various steps and methods described herein. By way of example, the memory can have instructions stored thereon causing the remote key authority to execute steps as shown in FIG. 3.

The remote key authority 132 can be configured to evaluate the request for permission received from and/or initiated by the external medical device programmer 116. In some embodiments, the remote key authority 132 can be configured to select between automatically responding and requesting human input from an authoritative system expert 152. For example, some restricted instructions may be "system determinable" in that the system can either authorize or deny permission to implement them based on the specific nature of the restricted instruction and facts about the patient and/or specific implanted device in question. Other restricted instructions may be "system non- determinable" in that the system must resort to seeking human input from an authoritative system expert 152 before being able to grant or deny permission for the restricted instructions.

If the remote key authority 132 grants permission to the external medical device programmer to implemented the restricted instruction, then the remote key authority 132 can effectuate this by generating a digital key, such as a cryptographic digital key, and sending the digital key to the external medical device programmer 116. The digital key can enable the external medical device programmer 116 to implement the restricted instructions on the implantable medical device. In some embodiments, the digital key can include a series of integers.

It will be appreciated that there are many ways in which to generate a digital key. For example, aspects of digital key generation can be found in U.S. Pat. No. 4,218,582. In some embodiments, the cryptographic digital key can include information derived from the verifying data originally passed from the external medical device programmer 116 to the remote key authority 132. For example, data from the verifying data can be used as a pass phrase and a key generation algorithm utilizing a cryptographic hash function such as SHA- 1 or SHA-2 can be used to generate the key. In some embodiments, the key can be generated with algorithm that uses, as input, data about the specific patient, information regarding the specific restricted instruction, and/or the current time of day.

In some embodiments, the cryptographic digital key can be effective to allow the external medical device programmer 116 to implement the restricted instructions on the implantable medical device only for a limited period of time, such as only for the current programming session or only for a predefined limited period of time. For example, in some embodiments, the cryptographic digital key can be effective to allow the external medical device programmer 116 to implement the restricted instructions on the implantable medical device only for as long as the current programming session between the external medical device programmer 116 and the implanted medical device 114. In some embodiments, the cryptographic digital key can be effective to allow the external medical device programmer 116 to implement the restricted instructions on the implantable medical device only for a predefined limited period of time. By way of example, in some embodiments the cryptographic digital key can be effective for a period of time less than or equal to 48 hours. In some embodiments the cryptographic digital key can be effective for a period of time less than or equal to 24 hours. In some embodiments the cryptographic digital key can be effective for a period of time less than or equal to 12 hours. In some embodiments the cryptographic digital key can be effective for a period of time less than or equal to 2 hours.

As described above, the set of all possible instructions that can be sent from an external medical device programmer to a specific implanted medical device can include both non-restricted and restricted instructions. Non-restricted instructions can include those instructions for which authorization is not required before implementation on the implantable medical device. Non-restricted instructions can include common instructions such as those relating to therapy (e.g., pacing mode, stimulation amplitude, pulse width, stimulation rate, and the like). Restricted instructions can include those instructions for which authorization is required before implementation on the implantable medical device. Restricted instructions include one or more of, system reset, firmware updates, reloading of software onto the implantable medical device. Restricted instructions can further include regulatory restricted and non-regulatory restricted instructions.

Regulatory restricted instructions are those that may require action relating to a regulatory body either prior to or after implementation of the instructions. For example, the implementation of some instructions may require the implantable device manufacturer to notify a regulatory body after implementation. As another example, the implementation of some instructions may require prior regulatory body approval before implementation.

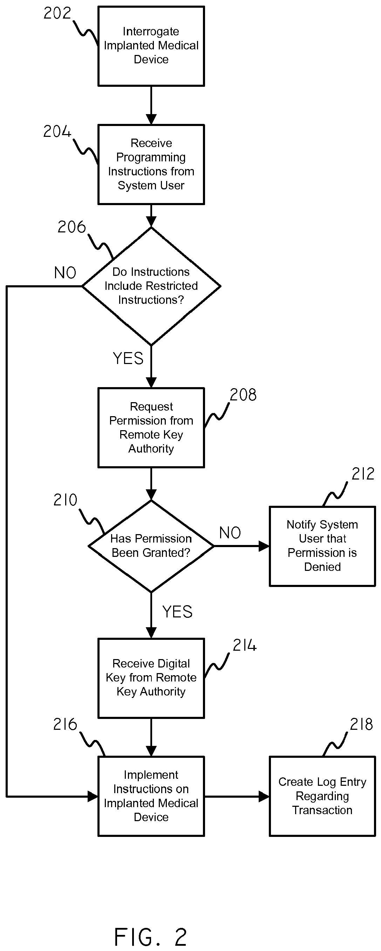

FIG. 2 is a flow diagram in accordance with at least one embodiment of the technology disclosed herein. In operation 202 an external medical device programmer can interrogate an implanted medical device. Interrogation can include establishing communication with the implanted medical device and querying and receiving information about the specific implanted medical device and/or the patient in which the implanted medical device lies. In another operation 204, the external medical device programmer can receive programming instructions from a system user. The programming instructions can be selected from a set of instructions including restricted and non-restricted instructions.

In another operation 206, the external medical device programmer can determine whether or not the instructions include restricted instructions. Though various techniques could be used, in at least one embodiment the external medical device programmer can determine whether or not the instructions include restricted instructions by querying each instruction against a database that indicates whether or not the particular instruction is restricted.

If the instructions do not include restricted instructions, then the system can proceed to another operation 216 wherein the instructions are implemented on an implanted medical device. Implementing the instructions on an implanted medical device can include physically sending the instructions to the implanted medical device or otherwise causing the instructions to take effect on the implanted medical device.

However, if the instructions do include restricted instructions, then in operation 208 the external medical device programmer can request permission from a remote key authority. After requesting permission, the external medical device programmer can determine whether or not permission has been granted in operation 210. If permission has not been granted, then the external medical device programmer can notify a system user that permission is denied in operation 212, ending the process. Notification can take place through a variety of ways such as a message on screen (e.g., a pop-up box, or the like), an audio cue, or the like. However, if permission has been granted, then in operation 214 the external medical device programmer can receive a digital key from a remote key authority. After receiving the digital key, the system can implement instructions on an implanted medical device in another operation 216. Finally, the external medical device programmer can create a log entry regarding the transaction in operation 218. The log entry can be stored locally on the external medical device programmer, on a computer that the external medical device programmer can communicate with, or pushed onto the implanted medical device itself.

FIG. 3 is a flow diagram in accordance with at least one embodiment of the technology disclosed herein. In operation 302 a remote key authority can receive a permission request regarding restricted instructions along with identifying information regarding the particular external medical device programmer sending or initiating the request, the particular implanted medical device, or the particular patient into which the medical device is implanted.

In operation 304 the remote key authority can determine whether or not the request is for system determinable instructions. Though various techniques could be used, in at least one embodiment the remote key authority can determine whether or not the request is for system determinable instructions by querying a database that indicates whether or not the restricted instructions are determinable based on the identity or type of the restricted instructions.

If the request is not for system determinable instructions, then the remote key authority can query an authoritative system expert in operation 306, before assessing whether permission should be granted in operation 308. However, if the request is for system determinable instructions, then the remote key authority can move onto assessing whether permission should be granted in operation 308 without querying an authoritative system expert.

If it is determined in operation 308 that permission should not be granted, then in operation 310 the remote key authority can send a notice to the external medical device programmer that permission is denied. However, if it is determined in operation 308 that permission should be granted, then in operation 312, the remote key authority can send a digital key to the external medical device programmer. In operation 314, the remote key authority can create a log entry regarding the transaction (e.g. the request and the response provided). The log entry can be stored locally on the remote key authority, on a computer that the remote key authority can communicate with, on the external medical device programmer, or on the implanted medical device itself.

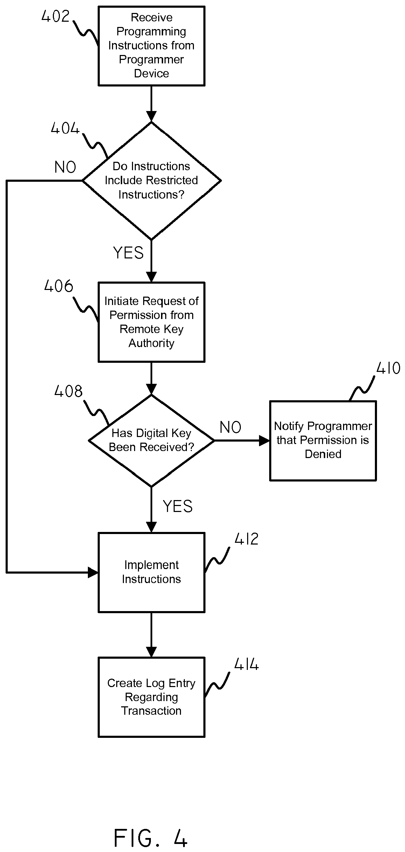

In some embodiments, the implanted medical device itself can initiate a request for permission from a remote key authority for a restricted instruction. For example, rather than the external medical device programmer serving as a gatekeeper and sending or initiating a request for permission based upon user input directing delivery of restricted instructions to the specific implanted medical device, the implanted medical device itself can initiate a request for permission from a remote key authority in some embodiments. FIG. 4 is a flow diagram in accordance with at least one embodiment of the technology disclosed herein. The implanted medical device can receive programming instructions from a programmer device in a first operation 402. The implanted medical device can then determine whether or not the instructions include restricted instructions in operation 404. If the instructions do not include restricted instructions then the implanted medical device can move on to implementing the instructions in operation 412.

However, if the instructions do include restricted instructions then the implanted medical device can initiate a request for permission from the remote key authority in operation 406. For example, initiating a request for permission from the remote key authority can include sending a notification back to the external medical device programmer that permission must be granted by a remote key authority before a specific instruction can be implemented. In some embodiments, initiating a request for permission from the remote key authority can include causing a request to be send directly to the remote key authority.

The implanted medical device can determine in operation 408 whether or not permission has been received from the remote key authority in the form of a digital key. If a digital key has not been received, then in operation 410 the implanted medical device can notify the external medical device programmer that permission has been denied regarding the restricted instruction. However, if a digital key has been received, then in operation 412 the implanted medical device can move on to implement the instructions. After implementing instructions, the implanted medical device can move on to create a log entry regarding the transaction in operation 414.

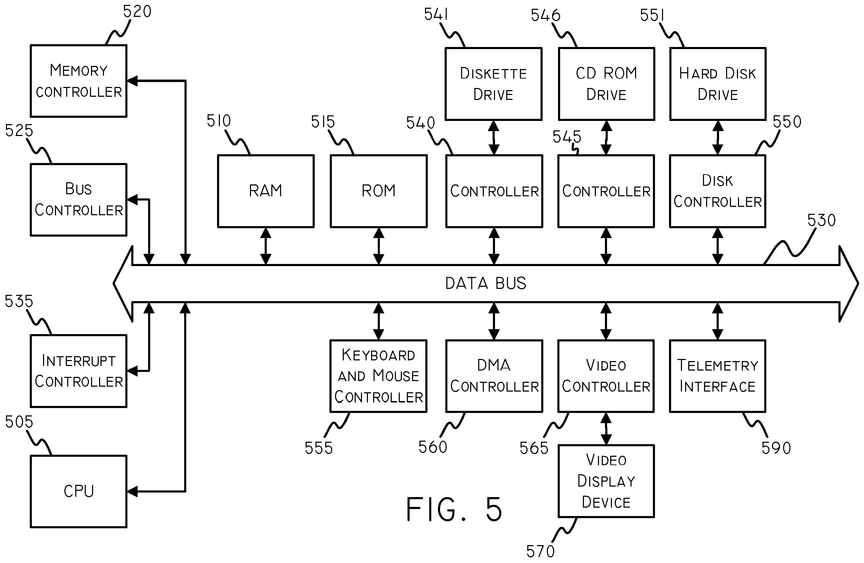

It will be appreciated that external medical device programmers of various embodiments can include a variety of specific components in order to carry out specific functions and methods as described herein. Referring now to FIG. 5, a diagram of various components that can be included in an external medical device programmer is shown in accordance with an embodiment of the invention. The system can include control circuitry including, for example, a central processing unit (CPU) 505 or processor, which may include a conventional microprocessor, random access memory (RAM) 510 for temporary storage of information, and read only memory (ROM) 515 for permanent storage of information. The memory (RAM and/or ROM) can have instructions stored thereon causing the external medical device programmer to execute various steps and methods described herein. By way of example, the memory can have instructions stored thereon causing the external medical device programmer to implement the steps according to FIG. 2. A memory controller 520 is provided for controlling system RAM 510. A bus controller 525 is provided for controlling data bus 530, and an interrupt controller 535 is used for receiving and processing various interrupt signals from the other system components.

Mass storage may be provided by diskette drive 541, which is connected to bus 530 by controller 540, CD-ROM drive 546, which is connected to bus 530 by controller 545, and/or a hard disk drive 551, which is connected to bus 530 by controller 550. User input to the system may be provided by a number of devices. For example, a keyboard and mouse can connected to bus 530 by keyboard and mouse controller 555. DMA controller 560 is provided for performing direct memory access to system RAM 510. A visual display is generated by a video controller 565, which controls video display 570. The system can also include a telemetry interface circuit 590 or wireless
communications module that allows the system to interface and exchange data with an implantable medical device. It will be appreciated that in various embodiments not all of the components depicted in FIG. 5 may be present.

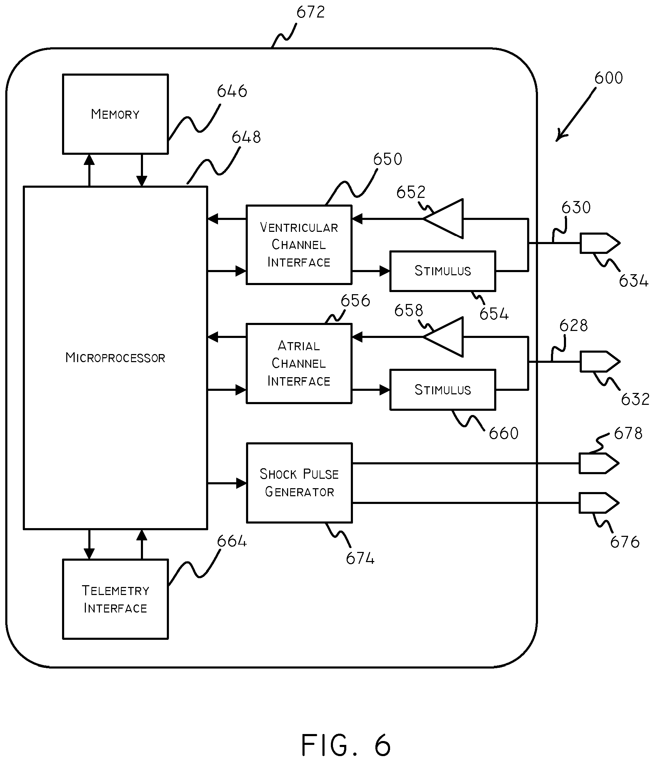

Referring now to FIG. 6, some components of an exemplary implantable system 600 are schematically illustrated. However, it will be appreciated that in some embodiments some of the elements of the controller module 672 shown in FIG. 6 may be omitted. Further, in some embodiments, additional elements may be included. The implantable medical system 600 can include a controller module 672 coupled to one or more stimulation leads 630 and 628. The controller module 672 can include a microprocessor 648 (or processor) that communicates with a memory 646. The memory 646 typically includes ROM or RAM for program storage and RAM for data storage. The controller module 672 can be configured to execute various operations such as execution of methods as described herein. A telemetry interface 664 is also provided for communicating with an external unit, such as a programmer device or a patient management system.

In some embodiments, the controller module 672 can include ventricular sensing and pacing channels including sensing amplifier 652, output circuit 654, and a ventricular channel interface 650 which communicates bidirectionally with a port of microprocessor 648. The ventricular sensing and pacing channel can be in communication with stimulation lead 630 and electrode 634.

In some embodiments, the controller module 672 can include atrial sensing and pacing channels including sensing amplifier 658, output circuit 660, and an atrial channel interface 656 which communicates bidirectionally with a port of microprocessor 648. The atrial sensing and pacing channel can be in communication with stimulation lead 628 and electrode 632. For each channel, the same lead and electrode can be used for both sensing and pacing.

The channel interfaces 650 and 656 can include analog-to-digital converters for digitizing sensing signal inputs from the sensing amplifiers and registers which can be written to by the microprocessor in order to output pulses, change the pacing pulse amplitude, and adjust the gain and threshold values for the sensing amplifiers. In some embodiments, a shock pulse generator 674 can also be interfaced to the microprocessor for delivering defibrillation shocks to the heart via a separate pair of electrodes 676, 678. In some embodiments, electrodes 676 and 678 can be disposed along stimulation lead 630 and stimulation lead 628 respectively.

It should be noted that, as used in this specification and the appended claims, the singular forms "a," "an," and "the" include plural referents unless the content clearly dictates otherwise. It should also be noted that the term "or" is generally employed in its sense including "and/or" unless the content clearly dictates otherwise.

It should also be noted that, as used in this specification and the appended claims, the phrase "configured" describes a system, apparatus, or other structure that is constructed or configured to perform a particular task or adopt a particular configuration. The phrase "configured" can be used interchangeably with other similar phrases such as "arranged", "arranged and configured", "constructed and arranged", "constructed", "manufactured and arranged", and the like.

One of ordinary skill in the art will understand that the modules, circuitry, and methods shown and described herein with regard to various embodiments of the invention can be implemented using software, hardware, and combinations of software and hardware. As such, the illustrated and/or described modules and circuitry are intended to encompass software implementations, hardware implementations, and software and hardware implementations. All publications and patent applications in this specification are indicative of the level of ordinary skill in the art to which this invention pertains.

This application is intended to cover adaptations or variations of the present subject matter. It is to be understood that the above description is intended to be illustrative, and not restrictive.

## Claims

1. A medical system comprising:
an external medical device programmer (116) comprising control circuitry and a wireless communications module for sending instructions selected from a set of instructions wirelessly to a specific implanted medical device (114), the set of instructions comprising restricted and non-restricted instructions, wherein restricted instructions include one or more of a system reset, a firmware update and reloading of software onto the implanted medical device (114); and
a remote key authority (132) configured to evaluate requests for permission received from the external medical device programmer (116);
the external medical device programmer (116) configured to receive input from a system user regarding which instructions from the set of instructions are to be delivered to the specific implanted medical device (114);
the external medical device programmer (116) configured to determine if the user input directs delivery of restricted or non-restricted instructions to the specific implanted medical device (114) and to request permission from the remote key authority (132) if the user input directs delivery of restricted instructions to the specific implanted medical device (114);
the remote key authority (132) configured to send a digital key to the external medical device programmer (116) if the remote key authority grants permission to the external medical device programmer (116); and
the external medical device programmer (116) configured to receive the digital key from the remote key authority (132) if permission is granted,
the digital key effective to allow the external programmer (116) to send restricted instructions to the specific implanted device (132), wherein the digital key is effective for a limited period of time.

2. The medical system of claim 1, wherein the external medical device programmer (116) is configured to interrogate the implanted medical device (114) and receives information about the specific implanted medical device (114) and about the patient into which the specific implanted medical device (114) has been implanted.

3. The medical system of claim 1 or claim 2, wherein the external medical device programmer (116) is configured to initiate a transfer of verifying data to the remote key authority (132), the verifying data including information about a patient into which the specific implanted medical device (114) has been implanted3, wherein the digital key including information derived from the verifying data and only effective to allow the external medical device programmer (116) to deliver restricted instructions to the specific implanted medical device (114).

4. The medical system of any of claims 1-3, the digital key comprising a cryptographic key.

5. The medical system of any of claims 1-4, the restricted instructions comprising a command to reset the implanted medical device (114).

6. The medical system of any of claims 1-5, the restricted instructions comprising regulatory restricted and non-regulatory restricted instructions.

7. The medical system of any of claims 1-6, the external medical device programmer (116) configured to distinguish between restricted and nonrestricted instructions.

8. The medical system of claim 1 further comprising: an implantable medical device (114).

9. The medical system of claim 8, the remote key authority (132) configured to select between automatically responding and requesting human input based on information regarding the restricted instruction.

10. The medical system of claim 8 or claim 9, restricted instructions comprising system determinable restricted instructions and system non- determinable restricted instructions, the remote key authority (132) configured to automatically respond to system determinable restriction instruction requests and request human input for system non-determinable restricted instructions.

11. A method for restricting programming instructions delivered to an implanted medical device comprising:
receiving (204) with an external medical device programmer (116) instructions from a system user for a specific implanted medical device (114);
determining (206) with the external medical device programmer (116) whether the instructions are restricted or non-restricted,
wherein restricted instructions include one or more of a system reset, a firmware update and reloading of software onto the implanted medical device,
sending (206) a request for permission from the external medical device programmer (116) to a remote key authority (132) if the instructions are restricted instructions;
evaluating (308) with the remote key the request for permission;
issuing (312) a digital key to the external medical device programmer (116) if permission is granted; and
receiving (214) a digital key from the remote key authority (132), the digital key configured to enable the external medical device programmer (116) to send the restricted instructions to the specific implanted medical device (114).

12. The method of claim 11, further comprising assessing with the external medical device programmer (116) whether the instructions are restricted or non-restricted.

13. The method of claim 11 or claim 12, further comprising interrogating the specific implanted medical device (114) and obtaining information about the specific implanted medical device (114) and the patient into which the implanted medical device (114) has been implanted.

14. The method of any of claims 11-13, further comprising recording data regarding the implementation of restricted instructions, the data including a time stamp

## Patentansprüche

1. Medizinisches System, umfassend:
eine externe Programmiereinrichtung (116) der medizinischen Vorrichtung, die Steuerschaltkreis(e) und ein Drahtloskommunikationsmodul zum Senden von Anweisungen ausgewählt aus einem Satz von Anweisungen in drahtloser Weise an eine spezifische implantierte medizinische Vorrichtung (114) umfasst, wobei der Satz von Anweisungen eingeschränkte und nicht eingeschränkte Anweisungen umfasst,
wobei die eingeschränkten Anweisungen ein oder mehrere von einem Reset des Systems, einer Firmware-Aktualisierung und einem erneuten Laden von Software auf die implantierte medizinische Vorrichtung (114) einschließen; und
eine Fernschlüsselstelle (132), die ausgestaltet ist, um Anfragen nach Genehmigung, die von der externen Programmiereinrichtung (116) der medizinischen Vorrichtung empfangen werden, auszuwerten;
wobei die externe Programmiereinrichtung (116) der medizinischen Vorrichtung ausgestaltet ist, um Eingabe von einem Systembenutzer diesbezüglich zu erhalten, welche Anweisungen von dem Satz von Anweisungen an die spezifische implantierte medizinische Vorrichtung (114) abgegeben werden sollen;
wobei die externe Programmiereinrichtung (116) der medizinischen Vorrichtung ausgestaltet ist, um zu ermitteln, ob die Benutzereingabe die Abgabe von eingeschränkten oder nicht eingeschränkten Anweisungen an die spezifische implantierte medizinische Vorrichtung (114) anweist, und um die Genehmigung von der Fernschlüsselstelle (132) anzufordern, falls die Benutzereingabe Abgabe von eingeschränkten Anweisungen an die spezifische implantierte medizinische Vorrichtung (114) anweist;
die Fernschlüsselstelle (132) ausgestaltet ist, um einen digitalen Schlüssel an die externe Programmiereinrichtung (116) der medizinischen Vorrichtung zu senden, falls die Fernschlüsselstelle der externen Programmiereinrichtung (116) der medizinischen Vorrichtung die Genehmigung erteilt; und
die externe Programmiereinrichtung (116) der medizinischen Vorrichtung ausgestaltet ist, um den digitalen Schlüssel von der Fernschlüsselstelle (132) zu empfangen, falls die Genehmigung erteilt wird,
wobei der digitale Schlüssel effektiv ist, um der externen Programmiereinrichtung (116) das Senden eingeschränkter Anweisungen an die spezifische implantierte Vorrichtung (132) zu erlauben, wobei der digitale Schlüssel für eine begrenzte Zeit effektiv ist.

2. Medizinisches System nach Anspruch 1, wobei die externe Programmiereinrichtung (116) der medizinischen Vorrichtung ausgestaltet ist, um die implantierte medizinische Vorrichtung (114) abzufragen, und Informationen zu der spezifischen implantierten medizinischen Vorrichtung (114) und zu dem Patienten empfängt, in den die spezifische implantierte medizinische Vorrichtung (114) implantiert worden ist.

3. Medizinisches System nach Anspruch 1 oder Anspruch 2, wobei die externe Programmiereinrichtung (116) der medizinischen Vorrichtung ausgestaltet ist, um eine Übertragung von Verifizierungsdaten an die Fernschlüsselstelle (132) zu initiieren, wobei die Verifizierungsdaten Informationen zu einem Patienten einschließen, in den die spezifische implantierte medizinische Vorrichtung (114) implantiert worden ist, wobei der digitale Schlüssel Informationen einschließt, die von den Verifizierungsdaten abgeleitet sind und nur effektiv sind, um der externen Programmiereinrichtung (116) der medizinischen Vorrichtung das Abgeben eingeschränkter Anweisungen an die spezifische implantierte medizinische Vorrichtung (114) zu erlauben.

4. Medizinisches System nach einem der Ansprüche 1 bis 3, wobei der digitale Schlüssel einen kryptographischen Schlüssel umfasst.

5. Medizinisches System nach einem der Ansprüche 1 bis 4, wobei die eingeschränkten Anweisungen einen Befehl zum Resetten der implantierten medizinischen Vorrichtung (114) umfassen.

6. Medizinisches System nach einem der Ansprüche 1 bis 5, wobei die eingeschränkten Anweisungen regulatorisch eingeschränkte und nicht regulatorisch eingeschränkte Anweisungen umfassen.

7. Medizinisches System nach einem der Ansprüche 1 bis 6, wobei die externe Programmiereinheit (116) der medizinischen Vorrichtung ausgestaltet ist, um zwischen eingeschränkten und nicht eingeschränkten Anweisungen zu unterscheiden.

8. Medizinisches System nach Anspruch 1, des Weiteren umfassend eine implantierbare medizinische Vorrichtung (114).

9. Medizinisches System nach Anspruch 8, wobei die Fernschlüsselstelle (132) ausgestaltet ist, um basierend auf Informationen, die die eingeschränkte Anweisung betreffen, zwischen automatischem Beantworten und Anfordern von menschlicher Eingabe auszuwählen.

10. Medizinisches System nach Anspruch 8 oder Anspruch 9, wobei die eingeschränkten Anweisungen vom System ermittelbare eingeschränkte Anweisungen und vom System nicht ermittelbare eingeschränkte Anweisungen umfassen, wobei die Fernschlüsselstelle (132) ausgestaltet ist, um automatisch auf vom System ermittelbare Einschränkungsanweisungs-anforderungen zu antworten und menschliche Eingabe für vom System nicht ermittelbare eingeschränkte Anweisungen anzufordern.

11. Verfahren zum Einschränken von Programmieranweisungen, die an eine implantierte medizinische Vorrichtung abgegeben werden, umfassend:
Empfangen (204) von Anweisungen von einem Systembenutzer für eine spezifische implantierte medizinische Vorrichtung (114) mit einer externen Programmiereinrichtung (116) der medizinischen Vorrichtung;
Ermitteln (206) mit der externen Programmiereinrichtung (116) der medizinischen Vorrichtung, ob die Anweisungen eingeschränkt oder nicht eingeschränkt sind,
wobei eingeschränkte Anweisungen einen oder mehrere von einem Reset des Systems, einer Firmware-Aktualisierung und erneutem Laden von Software auf die implantierte medizinische Vorrichtung einschließen,
Senden (206) einer Anforderung zur Genehmigung von der externen Programmiereinrichtung (116) der medizinischen Vorrichtung an eine Fernschlüsselstelle (132), falls die Anweisungen eingeschränkte Anweisungen sind;
Auswerten (308) der Anforderung zur Genehmigung mit dem Fernschlüssel;
Ausgeben (312) eines digitalen Schlüssels an die externe Programmiereinrichtung (116) der medizinischen Vorrichtung, falls die Genehmigung erteilt wird; und Empfangen (214) eines digitalen Schlüssels von der Fernschlüsselstelle (132), wobei der digitale Schlüssel ausgestaltet ist, um der externen Programmiereinheit (116) der medizinischen Vorrichtung das Senden der eingeschränkten Anweisungen an die spezifische implantierte medizinische Vorrichtung (114) zu erlauben.

12. Verfahren nach Anspruch 11, des Weiteren umfassend Beurteilen mit der externen Programmiereinheit (116) der medizinischen Vorrichtung, ob die Anweisungen eingeschränkt oder nicht eingeschränkt sind.

13. Verfahren nach Anspruch 11 oder Anspruch 12, des Weiteren umfassend Abfragen der spezifischen implantierten medizinischen Vorrichtung (114) und Erhalten von Informationen zu der spezifischen implantierten medizinischen Vorrichtung (114) und zu dem Patienten, in den die spezifische implantierte medizinische Vorrichtung (114) implantiert worden ist.

14. Verfahren nach einem der Ansprüche 11 bis 13, des Weiteren umfassend Aufzeichnen von Daten, welche die Implementierung von eingeschränkten Anweisungen betreffen, wobei die Daten einen Zeitstempel einschließen.

## Revendications

1. Système médical comprenant :
un programmateur externe de dispositif médical (116) comprenant un circuit de commande et un module de communication sans fil permettant d'envoyer des instructions sélectionnées à partir d'un ensemble d'instructions sans fil à un dispositif médical implanté spécifique (114), l'ensemble d'instructions comprenant des instructions limitées et non limitées, des instructions limitées comprenant une ou plusieurs opérations parmi une réinitialisation de système, une mise à jour de micrologiciel et un rechargement de logiciel sur le dispositif médical implanté (114) ; et
une autorité de clé à distance (132) configurée pour évaluer des demandes d'autorisation reçues à partir du programmateur externe de dispositif médical (116), le programmateur externe de dispositif médical (116) étant configuré pour recevoir une entrée en provenance d'un utilisateur de système au sujet duquel des instructions en provenance de l'ensemble d'instructions doivent être fournies au dispositif médical implanté spécifique (114) ;
le programmateur externe de dispositif médical (116) étant configuré pour déterminer si l'entrée utilisateur dirige la fourniture d'instructions limitées ou non limitées au dispositif médical implanté spécifique (114) et pour demander l'autorisation auprès de l'autorité de clé à distance (132) si l'entrée utilisateur dirige la fourniture d'instructions limitées au dispositif médical implanté spécifique (114) ;
l'autorité de clé à distance (132) étant configurée pour envoyer une clé numérique au programmateur externe de dispositif médical (116) si l'autorité de clé à distance accorde l'autorisation au programmateur externe de dispositif médical(l 16), et
le programmateur externe de dispositif médical(116) étant configuré pour recevoir la clé numérique de l'autorité de clé à distance (132) si l'autorisation est accordée,
la clé numérique étant effective pour permettre au programmateur externe (116) d'envoyer des instructions limitées au dispositif implanté spécifique (132), la clé numérique étant effective pendant une période de temps limitée.

2. Système médical selon la revendication 1, le programmateur externe de dispositif médical (116) étant configuré pour interroger le dispositif médical implanté (114), et recevant des informations à propos du dispositif médical implanté spécifique (114) et à propos du patient chez qui le dispositif médical implanté spécifique (114) a été implanté.

3. Système médical selon la revendication 1 ou la revendication 2, le programmateur externe de dispositif médical(116) étant configuré pour initier un transfert de données de vérification vers l'autorité de clé à distance (132), les données de vérification comprenant des informations à propos d'un patient chez qui le dispositif médical implanté spécifique (114) a été implanté, la clé numérique comprenant des informations dérivées des données de vérification, et étant effective uniquement pour permettre au programmateur externe de dispositif médical (116) de fournir des instructions limitées au dispositif médical implanté spécifique (114).

4. Système médical selon l'une quelconque des revendications 1 à 3, la clé numérique comprenant une clé cryptographique.

5. Système médical selon l'une quelconque des revendications 1 à 4, les instructions limitées comprenant une commande pour réinitialiser le dispositif médical implanté (114).

6. Système médical selon l'une quelconque des revendications 1 à 5, les instructions limitées comprenant des instructions limitées réglementaires et des instructions limitées non réglementaires.

7. Système médical selon l'une quelconque des revendications 1 à 6, le programmateur externe de dispositif médical (116) étant configuré pour distinguer entre des instructions limitées et des instructions non limitées.

8. Système médical selon la revendication 1, comprenant en outre : un dispositif médical implantable (114).

9. Système médical selon la revendication 8, l'autorité de clé à distance (132) étant configurée pour sélectionner entre une réponse automatique et une demande d'entrée humaine sur la base d'informations relatives à l'instruction limitée.

10. Système médical selon la revendication 8 ou la revendication 9, des instructions limitées comprenant des instructions limitées déterminables par le système et des instructions limitées non déterminables par le système, l'autorité de clé à distance (132) étant configurée pour répondre automatiquement aux demandes d'instructions limitées déterminables par le système et demander une entrée humaine pour des instructions limitées non déterminables par le système.

11. Procédé permettant de limiter des instructions de programmation fournies à un dispositif médical implanté comprenant :
la réception (204) avec un programmateur externe de dispositif médical (116) d'instructions en provenance d'un utilisateur de système pour un dispositif médical implanté spécifique (114),
la détermination (206) avec le programmateur externe de dispositif médical (116) du fait que les instructions sont limitées ou non-limitées,
des instructions limitées comprenant une ou plusieurs opérations parmi une réinitialisation de système, une mise à jour de micrologiciel et un rechargement de logiciel sur le dispositif médical implanté,
l'envoi (206) d'une demande d'autorisation à partir du programmateur externe de dispositif médical (116) à une autorité de clé à distance (132) si les instructions sont des instructions limitées ;
l'évaluation (308) avec la clé à distance de la demande d'autorisation ;
l'émission (312) d'une clé numérique au programmateur externe de dispositif médical (116) si l'autorisation est accordée ; et
la réception (214) d'une clé numérique en provenance de l'autorité de clé à distance (132), la clé numérique étant configurée pour permettre au programmateur externe de dispositif médical (116) d'envoyer les instructions limitées au dispositif médical implanté spécifique (114).

12. Procédé selon la revendication 11, comprenant en outre l'évaluation avec le programmateur externe de dispositif médical (116) du fait que les instructions sont limitées ou non limitées.

13. Procédé selon la revendication 11 ou la revendication 12, comprenant en outre l'interrogation du dispositif médical implanté spécifique (114) et l'obtention d'informations à propos du dispositif médical implanté spécifique (114) et du patient chez qui le dispositif médical implanté (114) a été implanté.

14. Procédé selon l'une quelconque des revendications 11 à 13, comprenant en outre l'enregistrement de données relatives à l'exécution des instructions limitées, les données comprenant un horodatage.
